(19) Europäisches Patentamt
European Patent Office
Office européen des brevets

(11) Publication number: **0 022 353**
**A1**

# (12) EUROPEAN PATENT APPLICATION

(21) Application number: 80302254.0

(22) Date of filing: 03.07.80

(51) Int. Cl.³: C 07 D 277/72
A 01 N 43/78

(30) Priority: 05.07.79 US 55104

(43) Date of publication of application:
14.01.81 Bulletin 81/2

(84) Designated Contracting States:
BE CH DE FR GB IT LI NL SE

(71) Applicant: MONSANTO COMPANY
Patent Department 800 North Lindbergh Boulevard
St. Louis, Missouri 63166(US)

(72) Inventor: D'Amico, John Joseph
1037 Darwick Court
Olivette Missouri 63132(US)

(74) Representative: Nash, Brian Walter et al,
Monsanto House 10-18 Victoria Street
London, SW1H 0NQ(GB)

## (54) Derivatives of 2-thioxo-3-benzothiazoline acetonitrile, process for their preparation and their use as leguminous plant growth regulants.

(57) The present invention relates to compounds of the formula

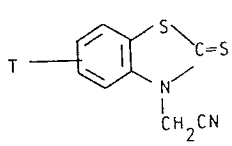

wherein T equals hydrogen, alkyl containing 1 to 5 carbon atoms, trifluoromethyl, hydroxy, chloro, bromo or fluoro. The invention also relates to a process for preparing said compounds as well as to the use of said compounds in a method of regulating leguminous plant growth and to plant growth regulant compositions.

EP 0 022 353 A1

Croydon Printing Company Ltd

0022353

TITLE MODIFIED
see front page



# DERIVATIVES OF 2-THIOXO-3-BENZOTHIAZOLINE ACETONITRILE AND THEIR USE AS LEGUMINOUS PLANT GROWTH REGULANTS

## Summary of the Invention

This invention is concerned with novel derivatives of 2-thioxo-3-benzothiazoline acetonitrile as well as to a novel method of preparing these compounds. It also relates to the use of these compounds in regulating the growth of leguminous plants and to the use of the novel compounds in plant growth regulant compositions.

## Background of the Invention

It is presently known that certain derivatives of 2-oxo-3-benzothiazoline acetonitrile possess both herbicidal and plant growth regulant activity. U.S. Patent No. 3,069,429 discloses the use of 4-halogeno-2-oxobenzothiazoline acetonitrile to kill weeds. U.S. Patent No. 3,993,468 discloses the use of 2-oxo-3-benzothiazoline acetonitrile to increase the yield of sucrose in sugar bearing plants and U.S. Patent No. 4,049,419 discloses the use of 2-oxo-3-benzothiazoline acetonitrile to increase the yield of harvestable leguminous plants. These patents do not, however, disclose the use of derivatives of 2-thioxo-3-benzothiazoline acetonitrile as effective, leguminous plant growth regulants.

U.S. Patent No. 3,050,526 discloses the preparation of 3-thiocyanomethyl-2-benzothiazolinones and 3-thiocyanomethyl-2-benzoxazolinones which are useful as fungicidal agents for the control of fungal diseases of plants.

None of the above-described prior art patents disclose the use of 2-thioxo-3-benzothiazoline acetonitrile and derivatives thereof, as leguminous plant growth regulants, nor would the teachings of these prior art patents enable one skilled in the art to prepare the novel 2-thioxo-3-benzothiazoline acetonitrile derivatives disclosed herein.

U.S. Patent No. 3,069,429, 3,993,468 and 4,049,419 teach the preparation of 2-oxo-3-benzothiazoline acetonitrile via a procedure which utilizes 2-oxo-benzothiazoline or 2-benzothiazolol as the starting material. Said starting material is reacted with chloroacetonitrile and potassium hydroxide or potassium carbonate in acetone and thereafter, the resulting mixture is heated under reflux for several hours. The reaction may be schematically illustrated as:

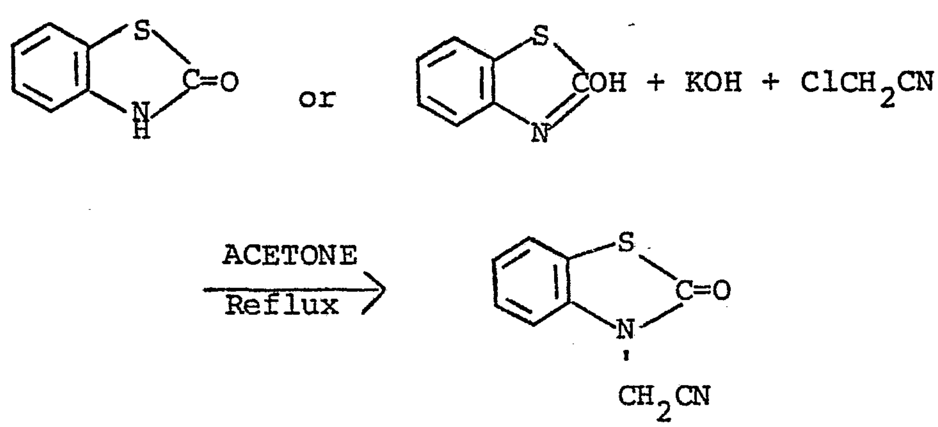


U.S. 3,993,468 and U.S. 4,049,419 also disclose the preparation of 2-oxo-3-benzothiazoline butyronitrile via the following reaction:

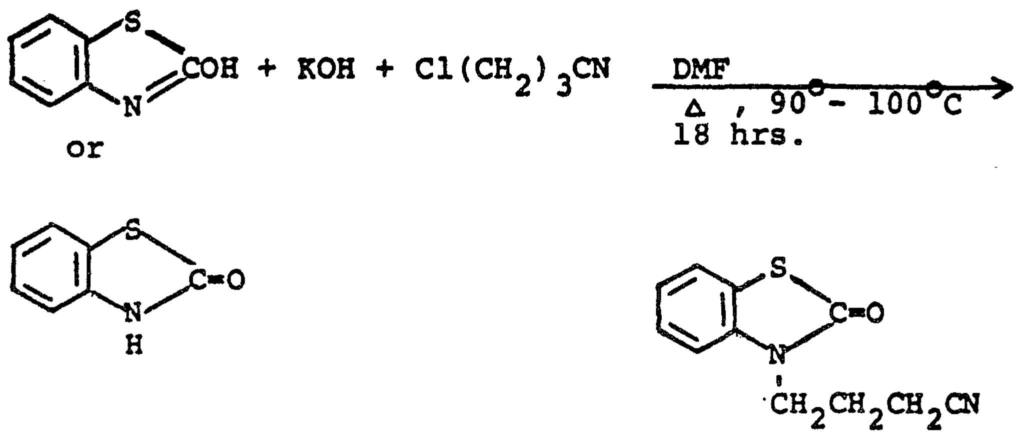


Although the above reaction appears quite general, it is well known (see, for example, C. H. Chen, ORG PREP AND PRO INT., 8(1), p. 1-2 (1976) that it is limited in that it is not applicable to the synthesis of 2-thioxo-3-benzothiazoline acetonitrile in nucleophilic reactions owing to the tautomeric nature of the

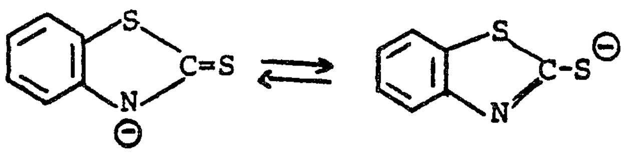 anion

which affords S- substitution almost exclusively under a variety of reaction conditions. For example, U.S. Serial No. 915,846, filed June 15, 1978, teaches the reaction of 2-mercaptobenzothiazole or 2-mercaptobenzoxazole with potassium hydroxide and chloroacetonitrile utilizing acetone as the solvent. The reaction may be schematically as:

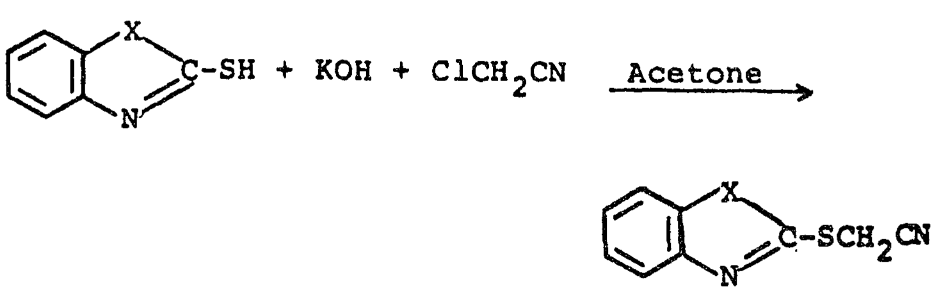


X = Sulfur or
Oxygen

In a similar fashion, when 2-mercaptobenzothiazole or 2-mercaptobenzoxazole is added to KOH and 4-chlorobutyronitrile and reacted in dimethylformamide (DMF) as the solvent, substitution occurs exclusively on the sulfur to produce compounds having the formula

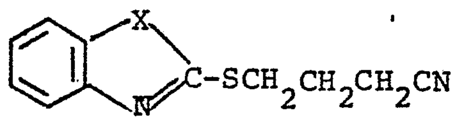

X = Oxygen or
    Sulfur.

U.S. Patent No. 3,050,526 teaches the preparation of 3-thiocyanomethyl-2-benzothiazolinones and 2-benzoxazolinones via the following reaction:

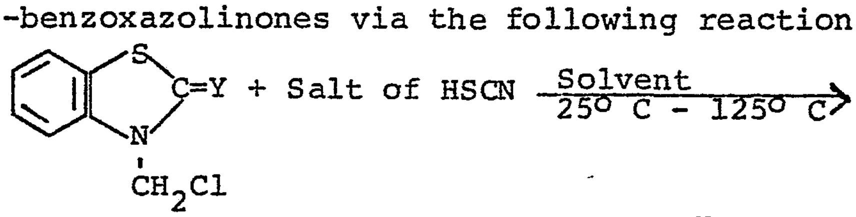


(x and y equal oxygen or sulfur but may not both equal sulfur simultaneously)

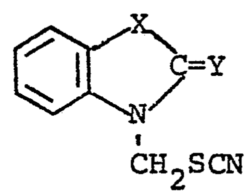

Mentioned as suitable solvents are acetone, methethyl ketone, benzene, toluene, lower alkanols, dimethylformamide, acetonitrile, ethylenedichloride and perchlorethylene. The examples shown in U.S, 3,050,526 utilize acetone as the solvent.

Surprisingly, attempts to synthesize derivatives of 2-thioxo-3-benzothiazoline acetonitrile according to the procedure of U.S. 3,050,526, utilizing acetone as the solvent, failed to yield the desired product, but, rather, the following reaction occurred:

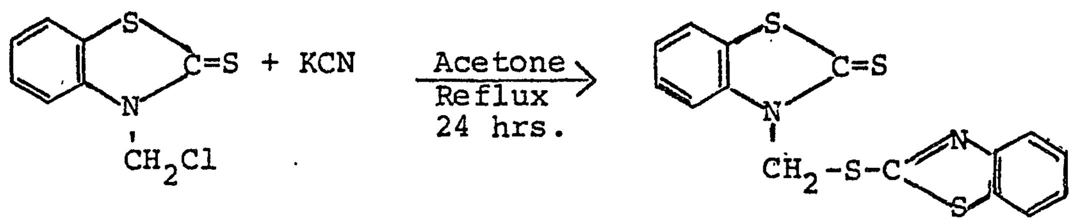


It has been found that, contrary to the above-described prior art teachings which teach no criticality of solvent in the synthesis of 2-oxo-3-benzothiazoline and-benzoxazoline acetonitrile, or 3-cyanothiomethyl-2-oxo-benzothiazoline and -benzoxazoline, the choice of solvent is critical in the practice of the process of the present invention.

The novel compounds of the present invention may be prepared according to the reaction illustrated below, only when dimethylformamide (DMF) or dimethylsulfoxide (DMSO) are utilized as the solvents. The reaction may be illustrated as:

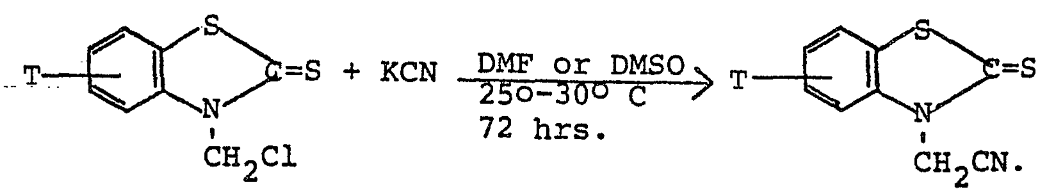


Accordingly, one object of the present invention is to provide a method of preparing novel 2-thioxo-3-benzothiazoline acetonitrile derivatives. Another object

of the invention is to describe the use of these novel 2-thioxo-3-benzothiazoline acetonitrile derivatives as leguminous plant growth regulators. Yet another object of the invention is to provide plant growth regulant compositions which comprise compounds of the invention in association with a diluent or carrier. The several embodiments of the invention are described in detail below.

Detailed Description of the Invention

The compounds employed in the present invention are 2-thioxo-3-benzothiazoline acetonitrile derivatives having the formula:

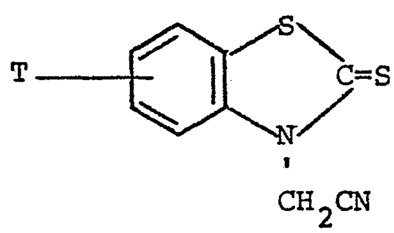

where T is hydrogen, alkyl containing 1 to 5 carbon atoms, hydroxy, trifluoromethyl, chloro, bromo or fluoro. It is preferred herein that T equal hydrogen.

Alkyl containing 1 to 3 carbon atoms is understood herein to refer to methyl, ethyl, n-propyl and isopropyl.

The novel compounds of the present invention may be prepared in accordance with the following reaction:

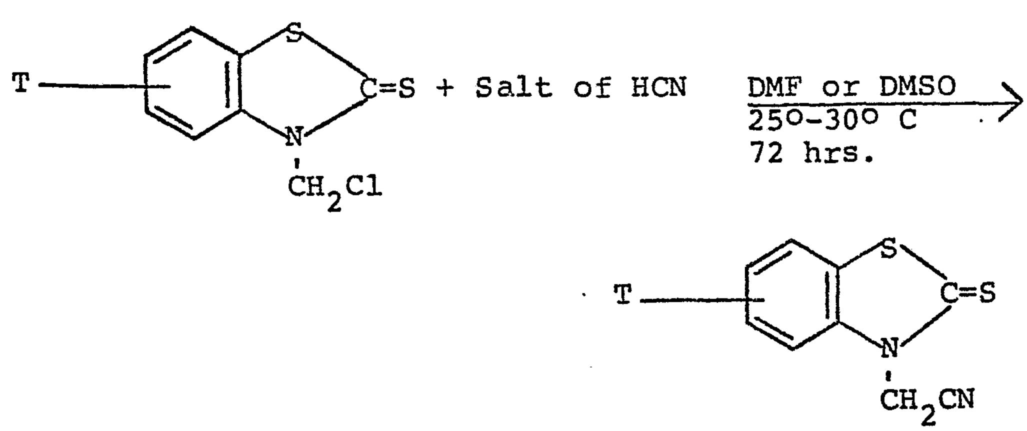

It will be recognized by those skilled in the art that there are a variety of available salts of HCN which may efficaciously be used in the above reaction. The potassium salt, i.e., KCN, is preferred for use herein.

The following illustrative example provides additional details of preparation.

Example 1

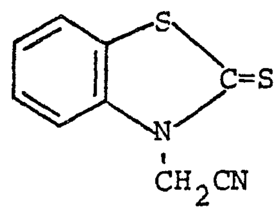

To a stirred slurry containing 71.6 g (1.1 mol) of potassium cyanide, one liter of dimethylformamide (DMF) and 90 ml of water, 215.8 g (1.0 mol) of 3-chloromethyl-2-benzothiazolenethione was added in one portion. After stirring for 25 minutes a temperature rise from 20° to 40° C was noted. After cooling to 25° C, the reaction mixture was stirred at 25°-30° C for 3 days. After the addition of 2 liters of water, stirring was continued at 25°-30° C for one hour. The solid was collected by filtration, washed with water until neutral to litmus and air-dried to 50° C. The crude product, mp 168°-170° C, was obtained in 98% yield. After recrystallization from toluene it melted at 179°-180° C.

Anal. Calc'd for $C_9H_6N_2S_2$: C,52.40; H,2.93; N,13.58; S,31.09;

Found: C,52.53; H,2.96; N,13.48; S,31.00.

Example 2

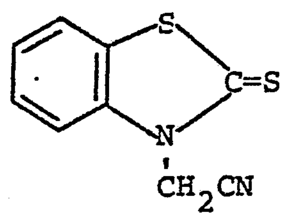

To a stirred slurry containing 71.6 g (1.1 mol) of potassium cyanide, one liter of dimethylsulfoxide (DMSO) and 90 ml of water, 215.8 g (1.0 mol) of 3-chloromethyl-2-benzothiazolenethione was added in one portion. After stirring for 25 minutes a temperature rise from 20° to 40° C was noted. After cooling to 25° C, the reaction mixture was stirred at 25°-30° C for 3 days. After the addition of 2 liters of water, stirring was continued at 25°-30° C for one hour. The solid was collected by filtration, washed with water until neutral to litmus and air-dried to 50° C. The crude product, mp 168°-170° C, was obtained in 98% yield. After recrystallization from toluene it melted at 179°-180° C.

Anal. Calc'd for $C_9H_6N_2S_2$: C,52.40; H,2.93; N,13.58; S,31.09;

Found: C,52.53; H,2.96; N,13.48; S,31.00.

As was previously discussed, attempts to prepare the 2-thioxo-3-benzothiazoline acetonitrile derivatives of the present invention, according to the procedure described in U.S. Patent No, 3,050,526 utilizing acetone as the solvent were unsuccessful. Example 3 describes in greater detail the procedure and the compound produced thereby when acetone was utilized as the solvent. Example 4 is the proof of structure of the compound prepared according to the procedure described in Example 3.

Example 3

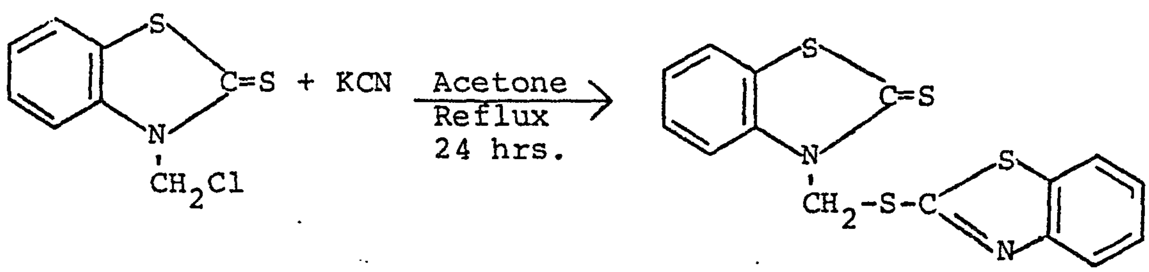


A stirred charge containing 21.6 g (0.1 mol) of 3-chloromethyl-2-benzothiazolinethione, 7.2 g (0.11 mol) of potassium cyanide in 150 ml of acetone was heated at reflux ($56^{o}$-$58^{o}$ C) for 24 hours. After cooling to $5^{o}$ C, 800 g of ice water was added and stirring continued at $0^{o}$-$10^{o}$ C for 30 minutes. The solid was collected by filtration, washed with water until neutral to litmus and air-dried at $25^{o}$-$30^{o}$ C. The crude product, mp $106^{o}$-$108^{o}$ C, was obtained in 92% yield. After recrystallization from toluene it melted at $130^{o}$-$131^{o}$ C.

Anal. Calc'd for $C_{15}H_{10}N_2S_4$: C,51.99;H,2.91;
N,8.08; S,37.01;
M.W. 346.5;

Found: C,52.09; H,2.96;
N,7.97; S,36.80;
M.W. 349.0 (DMF).

Example 4

(Proof of Structure of Example 3)

To a stirred solution containing 17 g (0.1 mol) of 2-mercaptobenzothiazole 6.6 g (0.1 mol) of 85% potassium hydroxide, 200 ml of acetone and 10 ml of water, 21.6 g (0.1 mol) of 3-chloromethyl-2-benzothiazolinethione was added in one portion. The stirred reaction mixture was heated at reflux (56°-58° C) for 24 hours. After cooling to 10° C, 800 ml of water was added and stirring continued at 25°-30° C for 30 minutes. The solid was collected by filtration, washed with water until neutral to litmus and air-dried at 25°-30° C. The crude product, mp 122°-124° C, was obtained in 81% yield. After recrystallization from toluene it melted at 131°-132° C. A mixture melting point with the product of Example 3 was not depressed and the nmr special data were identical.

Anal. Calc'd for $C_{15}H_{10}N_2S_4$: C,51.99; H,2.91; N,8.08; S,37.01;
Found: C,52.14; H,2.96; N,8.01; S,36.81.

As noted above, the compounds of the present invention have been found to be effective in the regulation of leguminous plant growth preferably the normal growth and development of soybean (Glycine max).

The terms "plant growth regulant effect", "plant growth regulation" or words to that effect, are used in this specification and in the claims to mean the causation by the chemicals of the present invention, of a variety of plant responses which achieve a promotion, inhibition or modification of any plant physiological or morphological process. It should additionally be recognized that various plant responses may also result from a combination or sequence of both physiological and morphological factors. Such plant

responses are most readily observed as changes in size, shape, color or texture of the treated plant or any of its parts. The above changes may be characterized as an acceleration or retardation of plant growth, stature reduction, leaf or canopy alteration, increased branching, increased fruit set, accelerated fruit set and the like. While many of these modifications are desirable in and of themselves, most often it is their effect on the economic result that is of most importance. For example, a reduction in stature of the plant permits the growing of more plants per unit area.

It is to be understood that the regulation of desirable leguminous crop plants in accordance with the instant invention does not include the total inhibition or the killing of such plants. Although phytotoxic amounts of the materials disclosed herein might be employed to exert a herbicidal (killing) action, it is contemplated here to employ only plant regulating amounts of such materials in order to modify the normal sequential development of the treated plant to agricultural maturity. The application of a plant regulating amount may be applied to plants in sequence at various stages of the plants' development to obtain various desirable responses. As may be expected, and as is apparent to those skilled in the art, such plant regulating amounts will vary, not only with the material selected, but also with the modifying effect desired, the species of plant and its stage of development, the plant growth medium and whether a permanent or transitory effect is sought.

In accordance with this invention, it has been found that desirable modification of leguminous crop plants is achieved by applying the above-described plant regulants to the "plant" or plant "habitat". The term "plant" is understood herein to include the seeds, emerging seedlings,

roots, stems, leaves, flowers, fruits or other plant parts. The term "habitat" is understood herein to mean the environment of the plant such as the plant growing medium, e.g., the soil.

In accordance with the practice of the invention, a plant growth regulating composition was formulated by utilizing 2-thioxo-3-benzothiazoline acetonitrile as the active ingredient in an acetone (containing Tween 20 surfactant) diluent. The composition thus formulated exhibited plant regulatory properties as illustrated by the test set forth in Example 5.

Example 5

A number of soybean plants, variety Williams, were grown from seeds in plastic pots in the greenhouse for a period of one week, at which time the plants were thinned to one plant per pot. When the second trifoliate leaf (three weeks) was fully expanded, the plants were treated with a solution of the active ingredient in acetone and water containing Tween 20 surfactant. When the fifth trifoliate leaf (four or five weeks) was fully expanded, the treated plants were compared with the non-treated control plants and the observations recorded.

Table I below summarizes the results and observations made in accordance with the above procedure.

Table I

| Compound of Example | Rate Lbs/Acre | Rate Kilos/Hectare | Response |
|---|---|---|---|
| 1* | 2.5 | 2.80 | Stem distortion, leaf alteration, leaf inhibition, leaf alteration new growth. |
| | 0.5 | 0.56 | Leaf inhibition, leaf alteration, leaf alteration new growth. |
| | 0.1 | 0.11 | Leaf inhibition, leaf alteration, leaf alteration new growth. |

*Data combined from four tests.

In selecting the appropriate time and rate of application of the active ingredient, it will be recognized that precise rates will also be dependent upon the desired response, mode of application, plant variety, soil conditions and various other factors known to those skilled in the art. While a rate of up to 11.2 kilos per hectare may be used, rates below 6.72 kilos per hectare are contemplated and rates from about 0.11 to about 5.60 kilos per hectare are preferred. In addition, it will be recognized that single or multiple applications may be used to exert the desired response.

In the practice of the invention, the active ingredient can be used alone or in combination with materials referred to in the art as adjuvants, in either liquid or solid form. To prepare such compositions, the active ingredient is admixed with an adjuvant including diluents, extenders, carriers and conditioning agents to provide compositions in the form of finely-divided particulate solids, granules, pellets, wettable powders, dusts, solutions and aquous dispersions or emulsions. Thus, the active ingredient can be used with an adjuvant such as a finely-divided particulate solid, a solvent liquid or organic origin, water, a wetting agent, dispersing agent or emulsifying agent or any suitable combination of these.

Illustrative finely-divided solid carriers and extenders which are useful in plant growth regulating compositions of this invention include talcs, clays, pumice, silica, diatomaceous earth, quartz, Fullers earth, sulfur, powdered cork, powdered wood, walnut flour, chalk, tobacco dust, charcoal and the like. Typical liquid diluents include Stoddard solvent, acetone, alcohols, glycols, ethyl acetate, benzene and the like. The plant growth regulating compositions of this invention, particularly liquids and wettable powders, usually contain one or more surface-active agents

in amounts sufficient to render a given composition readily dispersible in water or in oil. The term "surface-active agent" is understood to include wetting agents, dispersing agents, suspending agents and emulsifying agents. Such surface-active agents are well known and reference is made to U.S. Patent No, 2,547,724, columns 3 and 4, for detailed examples of the same.

Generally, the active ingredients are applied in the form of a composition containing one or more adjuvants which aid in the uniform distribution of the active ingredient. The application of liquid and particulate solid compositions of the active ingredient can be carried out by conventional techniques utilizing, for example, spreaders, power dusters, boom and hand sprayers and spray dusters. The composition can also be applied from airplanes as a dust or spray.

Compositions of this invention generally contain from about 5 to 95 parts active ingredient, about 1 to 50 parts surface-active agent and about 4 to 94 parts solvents, all parts being by weight based on the total weight of the composition.

Although this invention has been described with respect to specific modifications, the details thereof are not to be construed as limitations, for it will be apparent that various equivalents, changes and modifications may be resorted to without departing from the spirit and scope thereof and it is understood that such equivalent embodiments are intended to be included herein.

CLAIMS

1. A process for the preparation of a compound having the formula

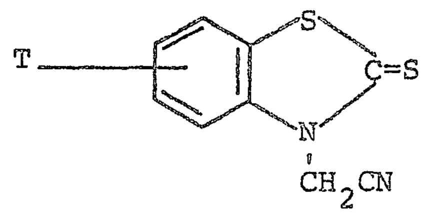

where T is equal to hydrogen, alkyl containing 1 to 5 carbon atoms, trifluoromethyl, hydroxy, chloro, bromo or fluoro, which comprises reacting, in the presence of a solvent selected from the group consisting of dimethylformamide or dimethylsulfoxide, a salt of HCN and a compound having the formula

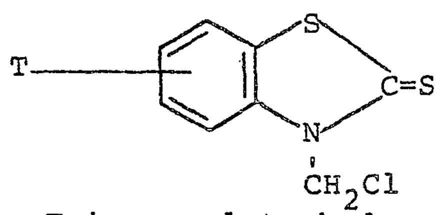

where T is equal to hydrogen, alkyl containing 1 to 5 carbon atoms, trifluoromethyl, hydroxy, chloro, bromo or fluoro.

2. A process according to Claim 1 wherein said salt of HCN is the potassium salt.

3. A process according to Claim 1 wherein said solvent is dimethylformamide.

4. A process according to Claim 1 wherein said solvent is dimethylsulfoxide.

5. A compound of the formula

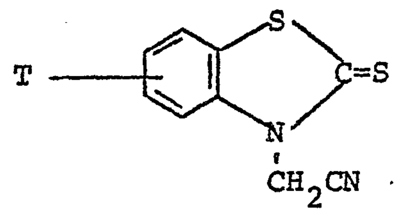

where T is equal to hydrogen, alkyl containing 1 to 5 carbon atoms, trifluoromethyl, hydroxy, chloro, bromo or fluoro.

6. A compound according to Claim 5 wherein T is hydrogen.

7. A method of regulating the natural growth and development of leguminous plants which method comprises applying to said leguminous plants or their habitat an effective plant growth regulating amount of a compound of the formula

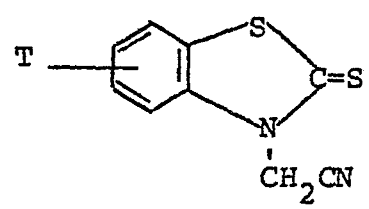

where T is hydrogen, alkyl containing 1 to 5 carbon atoms, trifluoromethyl, hydroxy, chloro, bromo or fluoro.

8. A method according to Claim 7 wherein T is hydrogen.

9. A method according to Claim 7 wherein said leguminous plants are soybean plants.

10. A plant growth regulating composition comprising an inert adjuvant and as the active ingredient from about 5 to about 95 parts by weight of an amount of a compound of the formula

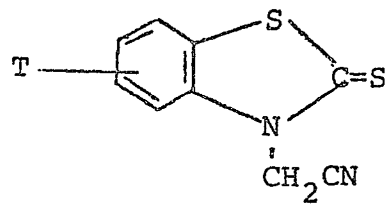

where T is hydrogen, alkyl containing 1 to 5 carbon atoms, trifluoromethyl, hydroxy, chloro, bromo or fluoro.

11. A plant growth regulating composition according to Claim 10 wherein T is hydrogen.

0022353

European Patent Office

# EUROPEAN SEARCH REPORT

Application number
EP 80 30 2254

## DOCUMENTS CONSIDERED TO BE RELEVANT

| Category | Citation of document with indication, where appropriate, of relevant passages | Relevant to claim |
|---|---|---|
| | CHEMICAL ABSTRACTS, Chemical Substance Index, vol. 88, 30th June 1978, page 909CS, column 3, no. 3(2H) Columbus, Ohio, U.S.A. "Benzothiazoleacetonitrile, α,α-dimethyl-2-thioxo" * Abstract * -- | 5 |
| | CHEMICAL ABSTRACTS, vol. 88, no. 21, 22nd May 1978, page 107, no. 146733e Columbus, Ohio, U.S.A. KH. G. ABDULLAEV et al.: "Antitrichomonadal activity of substances synthesized from benzimidazole and benzothiazoline" & DOKL. AKAD. NAUK UZB. SSR 1977, (6), 43-4 * Abstract and Chemical Substance Index * -- | |
| D | US - A - 3 993 468 (JOHN J. D'AMICO) * Claims * -- | 5,7-11 |
| D | US - A - 4 049 419 (JOHN. J. D'AMICO) * Claims * -- | 5,7-11 |
| D | US - A - 3 069 429 (DAVID H. GODSON) * Columns 1,2 * -- ./. | 5,7-11 |

CLASSIFICATION OF THE APPLICATION (Int. Cl. 3)

C 07 D 277/72
A 01 N 43/78

TECHNICAL FIELDS SEARCHED (Int.Cl. 3)

C 07 D 277/72
277/68
A 01 N 43/78

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 11-09-1980 | HENRY |

EPO Form 1503.1 06.78

0022353

European Patent Office

# EUROPEAN SEARCH REPORT

Application number
EP 80 30 2254

-2-

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| P | EP - A - 0 006 347 (MONSANTO)<br>* Claims 1,2,5,11 *<br>---- | 5,7-11 | |
| | | | TECHNICAL FIELDS SEARCHED (Int. Cl. 3) |